# EUROPÄISCHE PATENTSCHRIFT

(11) **EP 0 893 683 B1**
(45) Veröffentlichungstag und Bekanntmachung des Hinweises auf die Patenterteilung: **14.12.2005**
(21) Anmeldenummer: 98113013.1
(22) Anmeldetag: 13.07.1998
(51) Int. Cl.: G01N 15/06, B03C 1/28

(54) **Sensor zum Erfassen der Verunreinigung eines Fluids**
Sensor for detecting fluid contamination
Capteur pour détecter la contamination d'un fluide

(30) Priorität: 22.07.1997 DE 19731458
(43) Veröffentlichungstag der Anmeldung: 27.01.1999
(73) Patentinhaber: Brueninghaus Hydromatik GmbH, 89275 Elchingen (DE); HYDAC Electronic GmbH, 66128 Saarbrücken (DE)
(72) Erfinder: Seifert, Bodo-Siegfried, 89075 Ulm (DE); Adler, Bernhard, 89275 Elchingen (DE); Kluck, Albert, 66442 Blieskastel (DE)
(74) Vertreter: Körfer, Thomas

(56) Entgegenhaltungen:
- DE-A- 3 221 778
- GB-A- 2 195 263
- US-A- 2 429 920
- US-A- 3 553 672
- US-A- 5 179 346
- US-A- 5 264 832
- US-A- 5 402 113

## Beschreibung

Die Erfindung betrifft einen Sensor zum Erfassen der Verunreinigung eines in einem Strömungskanal strömenden Fluids mit Metallpartikeln.

Insbesondere in der Ölhydraulik tritt das Problem auf, daß hydraulische Bauteile, z. B. Hydropumpen und Hydromotoren, einem Verschleiß unterliegen und dadurch das Hydraulikfluid mit Metallpartikeln verunreinigt wird. Diese Metallpartikel können sich in Ventilen, in durch das Hydraulikfluid geschmierten Lagern oder anderen Bauteilen festsetzten und dort Schäden verursachen. Der erfindungsgemäße Sensor dient zum Erfassen der Verunreinigung bzw. des Verunreinigungsgrades eines Fluids, insbesondere eines Hydraulikfluids, mit derartigen Metallpartikeln. Der elektrische Widerstand zwischen zwei Polen eines elektrischen Anschluß des erfindungsgemäßen Sensors ist dabei von dem Verunreinigungsgrad des strömenden Fluids abhängig, so daß mit dem erfindungsgemäßen Sensor ein Relais oder eine elektrische Steuerschaltung angesteuert werden kann, um das Hydrauliksystem abzuschalten, sobald der Verunreinigungsgrad einen vorgegebenen Schwellwert überschritten hat.

Sensoren zum Erfassen der Verunreinigung eines strömenden Fluids sind in verschiedenen Bauformen bekannt. Die bekannten Sensoren sind jedoch konstruktiv relativ aufwendig ausgebildet und daher relativ teuer in der Herstellung.

Ein Sensor zum Erfassen von Verunreinigung von Schmieröl in einem Ölsumpf eines Flügzeugmotors ist aus der US 2,429,920 bekannt. In einer Ölablassschraube, die das Gehäuse des Sensors bildet ist eine magnetische Elektrode angeordnet. Die magnetische Elektrode besteht aus einem mit einem ersten elektrischen Kontakt verbundenen Kern und einem Kopf, der zu der zu untersuchenden Flüssigkeit gerichtet ist. Um den Kern herum ist eine Spule angeordnet, die mit einem zweiten elektrischen Kontakt und mit dem Gehäuse als Masse verbunden ist, und die der Erzeugung eines Magnetfelds für die Elektrode dient. Zwischen dem Gehäuse und der magnetischen Elektrode ist ein topfförmiger Isolator in einer entsprechenden Ausnehmung der Ölablassschraube angeordnet.

Der Erfindung liegt die Aufgabe zugrunde, einen Sensor zum Erfassen der Verunreinigung eines strömenden Fluids mit Metallpartikeln zu schaffen, der konstruktiv einfach ausgebildet und kostengünstig herstellbar ist sowie gegen das Fluid besonders gut abgedichtet ist.

Die Aufgabe wird durch die Merkmale des Anspruchs 1 gelöst.

Der Erfindung liegt die Erkenntnis zugrunde, daß ein Magnet von einem metallischen Gehäusekörper durch einen den Magnet radial umgebenden Isolationskörper isoliert sein kann, wobei der Magnet mit einem ersten Pol eines elektrischen Anschlusses und der metallische Gehäusekörper mit einem zweiten Pol eines elektrischen Anschlusses verbunden ist. Durch den Magnet werden die Metallpartikel des strömenden Fluids angesammelt. Sobald die Metallpartikel den Isolationskörper an der in den Strömungskanal ragenden Stirnfläche überbrücken, ändert sich der elektrische Widerstand zwischen den Polen des elektrischen Anschlusses. Zusätzliche isolierte Metallhülsen oder zusätzliche Isolationskörper sind an der in den Strömungskanal ragenden Stirnfläche nicht erforderlich. Ferner ist der Magnet in einen metallischen Metallaufnahmekörper eingefaßt und der Isolationskörper als Kunststoffspritzteil ausgebildet, der zwischen den Magnetaufnahmekörper und den ersten Gehäusekörper eingespritzt ist. Durch die Kunststoffspritzgußtechnik ist eine besonders kostengünstige Fertigung des Sensors gegeben.

Die Ansprüche 2 bis 11 betreffen vorteilhafte Weiterbildungen der Erfindung.

Entsprechend Anspruch 2 kann sich an dem ersten, metallischen Gehäusekörper ein zweiter aus einem Kunststoff ausgebildeter Gehäusekörper anschließen, welcher zumindest mit einem Teil des Isolationskörpers als einstückiges Kunststoffspritzteil ausgebildet sein kann. Dadurch ergibt sich ein besonders hoher Integrationsgrad des erfindungsgemäßen Sensors. Entsprechend Anspruch 3 kann der aus Kunststoff bestehende zweite Gehäusekörper einen Anschlußraum umschließen, in welchem elektrische Anschlußleitungen mit dem ersten Gehäusekörper und dem Magnetaufnahmekörper z. B. durch Löten oder elektrisch Schweißen verbunden sind.

Der zweite Gehäusekörper und insbesondere der von diesem umschlossene Anschlußraum kann gemäß Anspruch 4 von dem ersten Gehäusekörper mittels eines ersten Dichtrings abgedichtet sein, der in einer ringförmige Ausnehmung des ersten Gehäusekörpers eingelegt ist und an welchem der als Kunststoffspritzteil ausgebildete zweite Gehäusekörper dicht anliegt, indem ein angespritzter Vorsprung des zweiten Gehäusekörpers die Ausnehmung des ersten Gehäusekörpers zusammen mit dem ersten Dichtring vollständig ausfüllt. Auf diese Weise entsteht eine besonders wirksame Abdichtung des von dem zweiten Gehäusekörper umgebenen Anschlußraums und es wird verhindert, daß das teilweise unter einem sehr hohen Druck stehende Fluid in den Anschlußraum vordringt und dort Beschädigungen hervorruft.

Entsprechend Anspruch 5 kann der erste Gehäusekörper mit einem Gewinde versehen sein und der zweite Gehäusekörper kann eine Angriffsfläche für ein geeignetes Werkzeug aufweisen, mit welchem der Sensor in die Wandung des Strömungskanals eingeschraubt wird. Entsprechend Anspruch 6 können der erste Gehäusekörper und der Magnetaufnahmekörper Nuten und/oder Vorsprünge aufweisen, in welche an dem Isolationskörper vorgesehene Vorsprünge und/oder Vorsprünge eingreifen, um eine gute Verbindung zwischen dem Isolationskörper und dem Gehäusekörper bzw. dem Magnetaufnahmekörper zu gewährleisten.

Der Isolationskörper kann entsprechend Anspruch 7 als mehrteiliges Bauteil ausgebildet sein, das einen Isolationsring, eine sich daran axial anschließende Isolationshülse und einen zwischen dem Isolationsring und der Isolationshülse angeordneten Dichtring umfaßt. Dadurch wird die Abdichtung des Anschlußraums weiter verbessert und ein Vordringen des teilweise unter einem hohen Druck stehenden Fluids an der Grenzfläche zwischen dem Isolationskörper und dem Magnet bzw. dem Magnetaufnahmekörper einerseits und der Grenzfläche zwischen dem Isolationskörper und dem metallischen ersten Gehäusekörper wird wirkungsvoll verhindert. Dabei können der zweite Gehäusekörper und die Isolationshülse als Kunststoffspritzteil einteilig entsprechend Anspruch 8 ausgebildet sein. Entsprechend Anspruch 9 kann der Isolationsring axiale Nuten oder Bohrungen aufweisen. Dadurch entsteht ein Druckausgleich zwischen der dem Fluid zugewandten und der dem Fluid abgewandten Stirnseite des Isolationsrings und die Druckbelastung des solationsrings wird verringert.

Entsprechend Anspruch 10 kann die wirksame Breite des Isolationskörpers durch Einsetzen eines Metallrings variiert werden. Entsprechend Anspruch 11 können der erste und zweite Gehäusekörper durch ein Rändel oder eine Verzahnung miteinander verbunden sein.

Nachfolgend wird die Erfindung anhand von Ausführungsbeispielen unter Bezugnahme auf die Zeichnung näher beschrieben. Es zeigen:
- Fig. 1: ein erstes Ausführungsbeispiel des erfindungsgemäßen Sensors in einer teilweise geschnittenen Darstellung;
- Fig. 2: einen vergrößerten Ausschnitt aus Fig. 1 in einer teilweise geschnittenen Darstellung;
- Fig. 3: einen teilweisen Schnitt durch ein zweites Ausführungsbeispiel des erfindungsgemäß Sensors;
- Fig. 4: einen teilweisen Schnitt durch ein drittes Ausführungsbeispiel des erfindungsgemäßen Sensors;
- Fig. 5: einen teilweisen Schnitt durch ein viertes Ausführungsbeispiel des erfindungsgemäßen Sensors und
- Fig. 6: ein elektrisches Schaltschema zur Verdeutlichung der Erfindung.

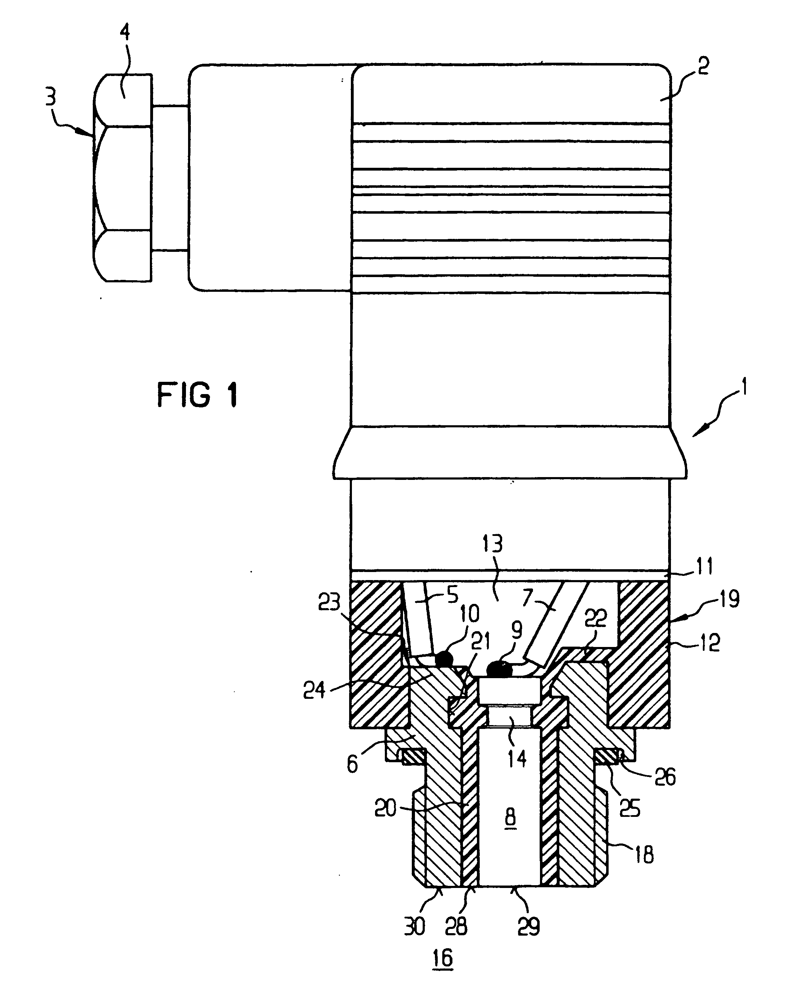

Fig. 1 zeigt ein erstes Ausführungsbeispiel des erfindungsgemäßen Sensors 1 in einer teilweise geschnittenen Darstellung. Ein Anschlußkörper 2 ist im Ausführungsbeispiel abgewinkelt ausgebildet und weist eine Öffnung 3 zur Einführung eines nicht dargestellten elektrischen Anschlußkabels auf. Das elektrische Anschlußkabel ist mittels einer Mutter 4 in der Öffnung 3 zur Zugentlastung arretierbar. Das in die Öffnung 3 eingeführte elektrische Anschlußkabel ist mit zumindest zwei nicht näher dargestellten Anschlußpolen des erfindungsgemäßen Sensors 1 verbunden. Einer der Pole eines elektrischen Anschlusses ist über eine isolierte Verbindungsleitung 5 mit einem ersten, metallischen Gehäusekörper 6 beispielsweise durch Löten oder Verschweißen verbunden. Eine zweite Verbindungsleitung 7 steht mit einem zweiten Pol des nicht dargestellten elektrischen Anschlusses des erfindungsgemäßen Sensors 1 in Verbindung und ist mit einem metallischen Magnetaufnahmekörper 8 ebenfalls z. B. durch Verlöten oder Verschweißen verbunden. Die Löt- oder Verschweißstellen sind durch die Bezugszeichen 9 und 10 angedeutet.

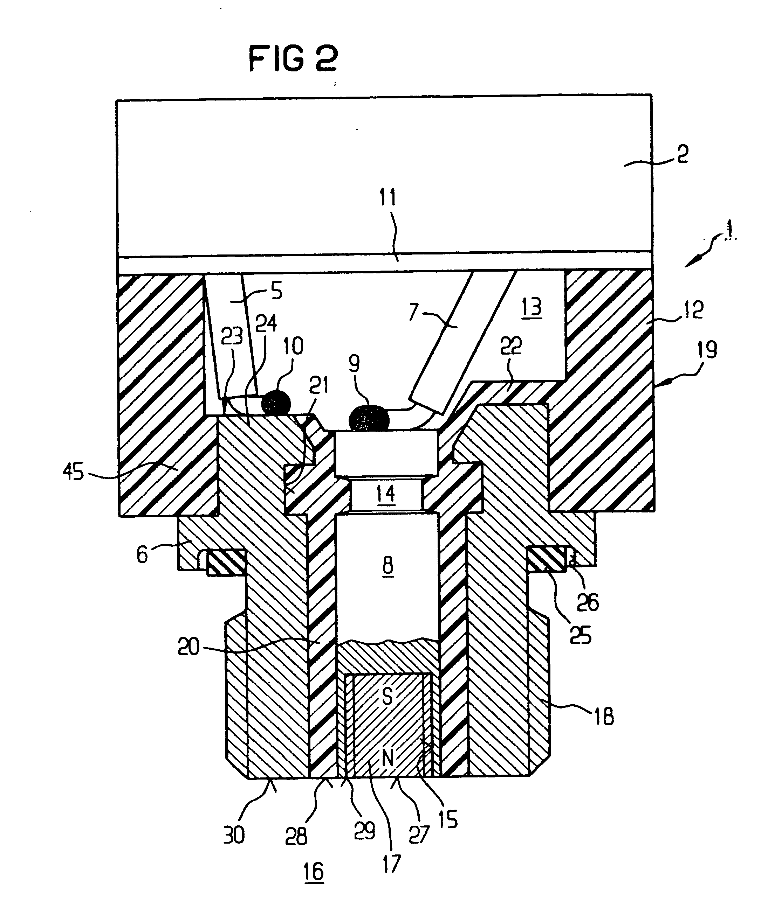

Der in Fig. 1 geschnitten dargestellte Bereich ist in Fig. 2 vergrößert dargestellt und kann daher aus dieser Figur besser ersehen werden. Zwischen dem abgewinkelten Anschlußkörper 2 und einem aus Kunststoff ausgebildeten, zwischen dem metallischen ersten Gehäusekörper 6 und dem Anschlußkörper 2 angeordneten zweiten Gehäusekörper 12 kann eine Dichtlippe 11 vorgesehen sein. Der zweite Gehäusekörper 12 umschließt einen Anschlußraum 13, in welchen die Verbindungsleitungen 5 und 7 aus dem Inneren des Anschlußkörpers 2 hineinragen und dort mit dem Magnetaufnahmekörper 8 bzw. dem ersten Gehäusekörper 6 vorzugsweise durch Verlöten oder Verschweißen verbunden sind.

Der Magnetaufnahmekörper 8 ist im dargestellten Ausführungsbeispiel als rotationssymmetrischer Stufenzylinder ausgebildet, der an seinem dem Anschlußraum 13 benachbartem Ende eine Einschnürung in Form einer Nut 14 aufweist. An seinem dem zu untersuchenden Fluid 16 zugewandten Ende weist der Magnetaufnahmekörper 8 eine Sackbohrung 15, vorzugsweise in Form einer Gewindebohrung, auf. In diese Sackbohrung 15 ist ein Permanentmagnet 17 eingesetzt, vorzugsweise eingeschraubt, so daß der Magnet 17 in den metallischen Magnetaufnahmekörper 8 eingefaßt ist.

Der erste, metallische Gehäusekörper 6 weist an seiner Mantelfläche einen Befestigungsabschnitt, vorzugsweise ein Gewinde 18 auf, um den Sensor 1 mit einem nicht weiter dargestellten Strömungskanal zu verbinden, in welchem das zu untersuchende Fluid 16 strömt. Im dargestellten Ausführungsbeispiel ist der Sensor 1 mittels des an dem ersten, metallischen Gehäusekörper 6 ausgebildeten Gewindes 18 in den Strömungskanal einschraubbar. Dazu weist der zweite, aus Kunststoff ausgebildete form- und kraftschlüssig mit dem ersten Gehäusekörper 6 verbundene zweite Gehäusekörper 12 eine entsprechende Angriffsfläche 19 auf, an welchem ein geeignetes Werkzeug, z. B. ein Maulschlüssel zum Einschrauben des Sensors 1 in eine Gewindebohrung des Strömungskanals angreifen kann. Die Angriffsfläche 19 kann insbesondere als Vierkant oder Sechskant ausgebildet sein.

Der Magnet 17 und der Magnetaufnahmekörper 8 sind von dem ersten, metallischen Gehäusekörper 6 durch einen Isolationskörper 20 elektrisch isoliert. Der Isolationskörper 20 ist vorzugsweise aus einem thermoplastischen Kunststoff gebildet und mittels eines Kunststoffspritzverfahrens als Kunststoffspritzteil zwischen den Magnetaufnahmekörper 8 und den metallischen, ersten Gehäusekörpers 6 eingespritzt. Zur axialen Arretierung weist der erste Gehäusekörper 6 zumindest eine Nut 21 auf, in welchen das Kunststoff des Isolationskörpers 20 genauso wie in die Nut 14 des Magnetaufnahmekörpers 8 während des Kunststoffspritzvorgangs eindringt. Somit ist eine axiale Fixierung des Magnetaufnahmekörpers 8 gegenüber dem Isolationskörper 20 und dem ersten Gehäusekörper 6 gewährleistet. Der Isolationskörper 20 ist bevorzugt mit dem zweiten Gehäusekörper 12 als einteiliges Kunststoffspritzteil ausgebildet und mit diesem durch einen umlaufenden Kragen 22 verbunden. Der Kragen 22 weist lediglich in einem geringen Winkelbereich eine Aussparung 23 auf, in welchem das dem Magneten 17 abgewandte Ende 24 des ersten, metallischen Gehäusekörpers 6 blank an den Anschlußraum 13 angrenzt. In diesem Bereich kann daher die Löt- oder Schweißverbindung mit der Verbindungsleitung 5 hergestellt werden. Durch die einstückige Ausbildung des zweiten Gehäusekörpers 12 mit dem Isolationskörper 20 entsteht ein besonders hoher Integrationsgrad des erfindungsgemäßen Sensors 1 und der Sensor 1 ist daher in einem besonders rationellen Fertigungsverfahren herstellbar.

Zur Abdichtung gegenüber der Aufnahmebohrung des nicht weiter dargestellten Strömungskanals ist eine Dichtung 25 vorgesehen, die in eine umlaufende Nut 26 des ersten Gehäusekörpers 6 eingesetzt ist.

Um eine besonders plane Oberfläche zu erhalten, können die Stirnfläche 27 des Magneten 17, die Stirnfläche 29 des Magnetaufnahmekörpers 8, die Stirnfläche 28 des Isolationskörpers 20 und die Stirnfläche 30 des ersten Gehäusekörpers 6 nach der Montage überschliffen werden.

Die Funktion des erfindungsgemäßen Sensors 1 ist folgendermaßen: Durch den Permanentmagneten 17 sammeln sich im Bereich seiner dem Fluid 16 zugewandten Stirnfläche 27 in dem Fluid 16 vorhandene Metallpartikel an. Aufgrund des magnetischen Streufeldes zwischen dem mit N gekennzeichneten magnetischen Nordpol und dem mit S gekennzeichneten magnetischen Südpol des als Stabmagnet ausgebildeten Magneten 17 verstreuen sich die Metallpartikel auch im Umgebungsbereich des Magneten 17, insbesondere in dem Bereich der in den Strömungskanal ragenden Stirnfläche 28 des Isolationskörpers 20. Wenn sich in diesem Bereich hinreichend viele Metallpartikel angesammelt haben, stellen diese eine leitende Überbrückung des Isolationskörpers 20 dar und verbinden den Magneten 17 bzw. den Magnetaufnahmekörper 8 mit dem ersten Gehäusekörper 6 elektrisch leitend bzw. vermindert zumindest den elektrischen Widerstand zwischen den nicht dargestellten Polen des elektrischen Anschlusses des Sensors 1 mit welchem die Verbindungsleitungen 5 und 7 in Verbindung stehen. Diese Widerstandsänderung wird als Meßsignal ausgenutzt und kann z. B. zur Ansteuerung eines elektrischen Relais oder einer anderen elektrischen Auswerteschaltung dienen. Selbstverständlich können der magnetische Nordpol N und Südpol S auch vertauscht sein.

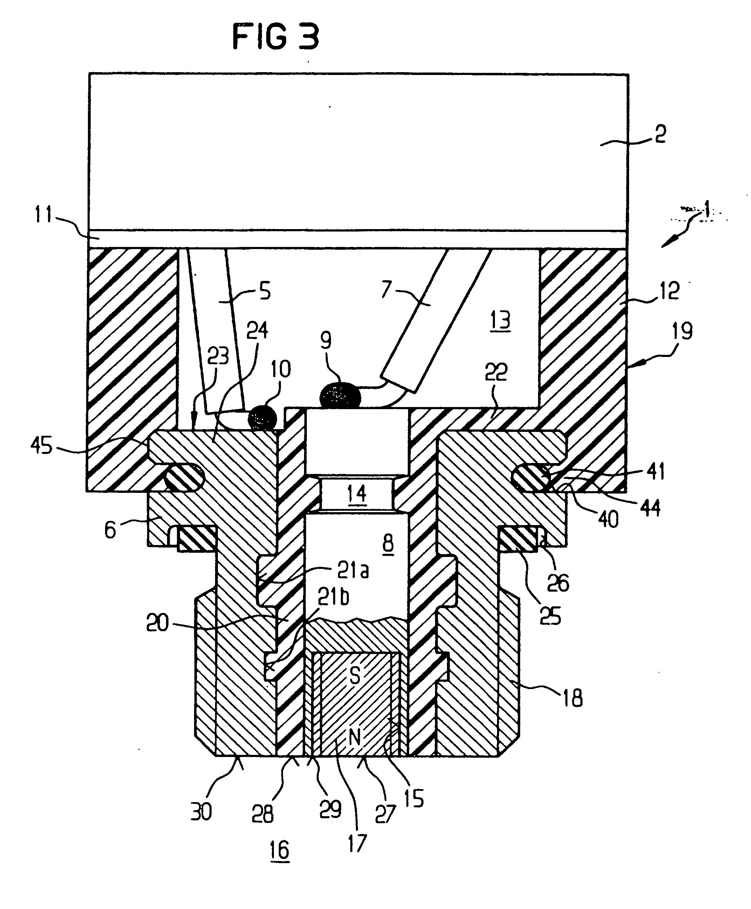

In Fig. 3 ist ein zweites Ausführungsbeispiel des erfindungsgemäßen Sensors 1 in einer auszugsweisen, geschnittenen Darstellung wiedergegeben. Der Ausschnittsbereich stimmt mit jenem der Fig. 2 überein und die übrigen Teile des Sensors können z. B. wie in Fig. 1 dargestellte ausgebildet sein. Bereits beschriebene Elemente sind mit übereinstimmenden Bezugszeichen versehen, so daß sich insofern eine wiederholende Beschreibung erübrigt.

Das in Fig. 3 dargestellte Ausführungsbeispiel unterscheidet sich von dem bereits anhand der Fig. 1 und 2 beschriebenen Ausführungsbeispiel im wesentlichen dadurch, daß an dem ersten, metallischen Gehäusekörper 6 eine ringförmige Ausnehmung 40 vorgesehen ist, in welcher ein erster Dichtring 41 vorzugsweise aus einem elastischen Material, wie Gummi, eingelegt ist. Das Einlegen des Dichtrings 41, z. B. eines O-Rings, erfolgt vor dem Ausbilden des den Isolationskörper 20 und den zweiten Gehäusekörper 12 umfassenden Kunststoffspritzteils. Der Kunststoff des zweiten Gehäusekörpers 12 dringt bei dem Kunststoffspritzverfahren daher als Vorsprung 44 in die Ausnehmung 40 ein und schließt die Ausnehmung 40 dichtend ab, indem sich der Vorsprung 44 an den ersten Dichtring 41 eng anlegt und die Ausnehmung 40 zusammen mit dem ersten Dichtring 41 vollständig ausfüllt. Dadurch wird eine besonders wirksame Abdichtung des Sensors 1, insbesondere des Anschlußraums 13 erreicht und verhindert, daß Feuchtigkeit in den Anschlußraum 13 vordringt und dort z. B. zu Korrosionen führt. Der erste Gehäusekörper 6 ist mit dem zweiten Gehäusekörper 12 durch ein Rändel 45 bzw. eine Verzahnung verbunden, so daß sich eine sichere Kraftübertragung zwischen den Gehäusekörpern 6 und 12 ergibt.

Des weiteren unterscheidet sich das in Fig. 3 dargestellte Ausführungsbeispiel von dem in den Fig. 1 und 2 dargestellten Ausführungsbeispiel dadurch, daß nicht nur eine radiale Nut 21 sondern zwei radiale Nuten 21a und 21b an dem Umfang des Isolationskörpers 20 vorgesehen sind.

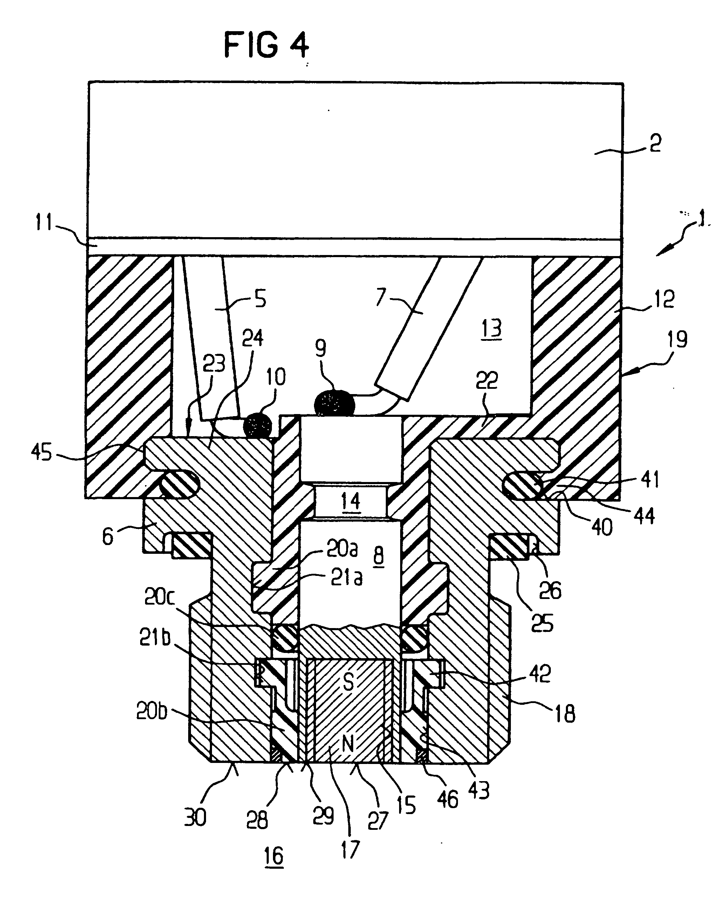

In Fig. 4 ist ein drittes Ausführungsbeispiel des erfindungsgemäßen Sensors 1 dargestellt. Bereits beschriebene Elemente sind mit übereinstimmenden Bezugszeichen versehen, so daß sich insoweit eine wiederholende Beschreibung erübrigt.

Das in Fig. 4 dargestellte Ausführungsbeispiel unterscheidet sich von dem in den Fig. 2 und 3 dargestellten Ausführungsbeispiel im wesentlichen dadurch, daß der Isolationskörper 20 mehrteilig ausgebildet ist. Der Isolationskörper 20 umfaßt einen dem zu untersuchenden Fluid 16 zugewandten Isolationsring 20b dessen axiale Erstreckung vorzugsweise mit der axialen Erstreckung des Magnets 17 im wesentlichen übereinstimmt, eine sich daran axial anschließende Isolationshülse 20a und einen zwischen der Isolationshülse 20a und dem Isolationsring 20b angeordneten zweiten Dichtring 20c, z. B. in Form eines O-Rings aus einem elastischen Material, z. B. Gummi. Die Isolationshülse 20a ist als Kunststoffspritzteil vorzugsweise einteilig mit dem zweiten Gehäusekörper 12 ausgebildet. Der Isolationsring 20b ist vorzugsweise ebenfalls als Kunststoffspritzteil ausgebildet. Die Isolationshülse 20a ist vorzugsweise in den Zwischenraum zwischen dem Magnetaufnahmekörper 8 und dem ersten Gehäusekörper 6 eingespritzt, so daß der Magnetaufnahmekörper 8 und der Gehäusekörper 6 die Kunststoffspritzform für die Isolationshülse 20a bilden. Dabei kann der Dichtring 20c vor dem Einspritzen des Kunststoffs für die Isolationshülse 20a eingesetzt werden, um einerseits den Abstand zwischen dem Magnetaufnahmekörper 8 und dem ersten Gehäusekörpers 6 zu fixieren und andererseits den Zwischenraum zwischen dem Magnetaufnahmekörper 8 und dem ersten Gehäusekörper 6 in Fig. 4 nach unten abzudichten. Dabei ergibt sich ferner eine besonders gute Dichtung, wie dies bereits in bezug auf den ersten Dichtring 41 beschrieben wurde.

In gleicher Weise kann auch der Isolationsring 20b in den Zwischenraum zwischen den Magnetaufnahmekörper 8 und dem ersten Gehäusekörper 6 eingespritzt werden. Vorzugsweise jedoch wird der Isolationsring 20b in einer Kunststoffspritzform außerhalb des Sensors 1 hergestellt und als vorgefertigtes Bauteil in den Zwischenraum zwischen dem Magnetaufnahmekörper 8 und dem ersten Gehäusekörper 6 eingesetzt. Dabei weist der Isolationsring 20b vorzugsweise eine Rastnase 42 auf, die in die ringförmige Nut 21b des ersten Gehäusekörpers 6 rastend eingreift. Der Isolationsring 20b kann dabei nicht dargestellte axiale Nuten oder axiale Bohrungen aufweisen, um einen Druckausgleich zwischen der dem Fluid 16 zugewandten Stirnfläche 28 und der gegenüberliegenden Stirnfläche des Isolationsrings 20b zu gewährleisten und zu verhindern, daß der Isolationsring 20b durch den teilweise sehr hohen Druck des Fluids 16 in Fig. 4 axial nach oben gedrückt wird.

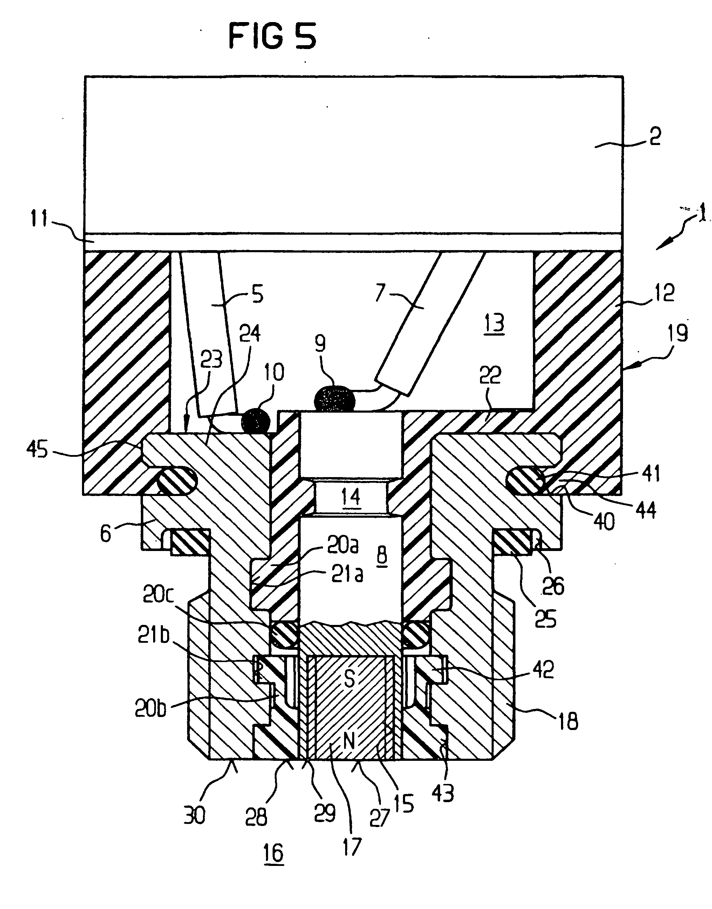

Wie an dem in Fig. 5 dargestellten Ausführungsbeispiel im Vergleich zu dem in Fig. 4 dargestellten Ausführungsbeispiel verdeutlicht, hat die separate Ausbildung des Isolationsrings 20b den Vorteil, daß der erfindungsgemäße Sensor 1 ohne Veränderung des konstruktiven Grundkonzepts mit Isolationsringen 20b unterschiedlicher Dicke an der Stirnfläche 28 ausgebildet werden kann. Bei der Fertigung ist dazu lediglich der Durchmesser der Bohrung 43 an dem dem Fluid 16 zugewandten Ende entsprechend anzupassen, so daß ein passender Isolationsring 20b in die Bohrung 43 einsetzbar ist. Eine Variation der Dicke des Isolationsrings 20b im Bereich der Stirnfläche 28 kann notwendig sein, um den erfindungsgemäßen Sensor 1 für unterschiedliche Anwendungen anzupassen, wobei von Anwendung zu Anwendung die Größe der Metallpartikel oder die Auslöseschwelle des Sensors 1 variieren kann. Alternativ ist bei dem Ausführungsbeispiel der Fig. 4 vorgesehen, einen metallischen Ring 46 in den Isolationsring 20b des Isolationskörpers einzusetzen und somit die Stirnfläche 28 je nach Breite des Metallrings 46 zu verschmälern. Dabei ist lediglich die Breite einer den Metallring 46 aufnehmenden Ausnehmung des Isolationsrings entsprechend anzupassen.

In Fig. 5 sind die übrigen, bereits beschriebenen Elemente mit übereinstimmenden Bezugszeichen versehen, so daß sich insoweit eine wiederholende Beschreibung erübrigt.

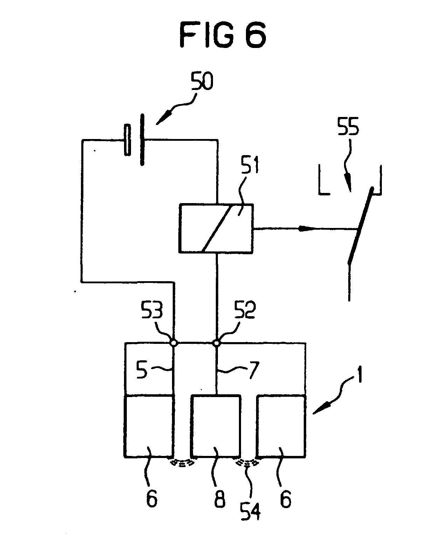

In Fig. 6 ist zum verbesserten Verständnis der Erfindung das elektrische Schaltschema für den erfindungsgemäßen Sensor 1 in einer vereinfachten Darstellung wiedergegeben. Eine elektrische Spannungsquelle 50 ist über ein elektrisches Relais 51 mit einem ersten Pol 52 des elektrischen Anschlusses des Sensors 1 verbunden. Der erste Pol 52 des elektrischen Anschlusses des Sensors 1 ist, wie bereits beschrieben, über eine interne Verbindungsleitung 7 mit dem Magnetaufnahmekörper 8 und somit mit dem Magnet 17 verbunden. Der andere Pol der elektrischen Spannungsquelle 50 ist über den zweiten Pol 53 des elektrischen Anschlusses des erfindungsgemäßen Sensors 1 und die interne Verbindungsleitung 5 mit dem ersten, metallischen Gehäusekörper 6 verbunden. Zwischen dem Magnet 17 bzw. dem Magnetaufnahmekörper 8 und dem ersten Gehäusekörper 6 mit dem Bezugszeichen 54 schematisch angedeutete Metallpartikel verringern der Widerstand zwischen den Polen 52 und 53 des elektrischen Anschlusses des erfindungsgemäßen Sensors 1. Wenn sich der Widerstand zwischen den Polen 52 und 53 unterhalb eines vorgegebenen Schwellwerts verringert, wird das elektrische Relais 51 betätigt. Dieses betätigt seinerseits einen elektrischen Schalter 55 oder ein Hydraulikventil. Daraufhin wird das hydraulische System automatisch abgeschaltet und vor einem Schaden durch die Metallpartikel 54 bewahrt.

Die Erfindung ist nicht auf die dargestellten Ausführungsbeispiele begrenzt. Die konstruktive Ausbildung des Sensors 1 ist auch in anderer Weise möglich. Insbesondere kann der Permanentmagnet 17 auch durch einen Elektromagneten ersetzt werden, bei dessen Ausschalten die Stirnfläche 27 des Magneten 17 ggf. unter Mitwirkung des strömenden Fluids 16 gereinigt wird, bevor ein neuer Meßintervall beginnt.

## Patentansprüche

1. Sensor (1) zum Erfassen der Verunreinigung eines in einem Strömungskanal strömenden Fluids (16) mit Metallpartikeln (54) mit
einem Magnet (17), welcher mit einem ersten Pol (52) eines elektrischen Anschlusses verbunden ist,
einem den Magnet (17) radial umgebenden Isolationskörper (20; 20a, 20b, 20c) und
einem den Isolationskörper (20; 20a, 20b, 20c) radial umgebenden, metallischen ersten Gehäusekörper (6), welcher durch den Isolationskörper (20; 20a, 20b, 20c) von dem Magnet (17) elektrisch isoliert ist und mit einem zweiten Pol (53) eines elektrischen Anschlusses verbunden ist und dessen Mantelfläche einen Befestigungsabschnitt (18) zum Befestigen des Sensors (1) an einer Wandung des Strömungskanals aufweist,
wobei die Stirnflächen (27, 28, 30) des Magneten (17), des Isolationskörpers (20; 20a, 20b, 20c) und des ersten Gehäusekörpers (6) im an den Strömungskanal befestigten Zustand in den Strömungskanal ragen und sich der elektrische Widerstand zwischen den Polen (52, 53) des elektrischen Anschlusses durch sich an dem Magnet (17) ansammelnde, den Isolationskörper (20; 20a, 20b, 20c) überbrückende Metallpartikel. (54) verringert,
wobei der Magnet (17) in einen metallischen Magnetaufnahmekörper (8) eingefaßt ist und
wobei der Isolationskörper (20) oder eine Isolationshülse (20a) eines mehrteiligen Isolationskörpers (20a, 20b, 20c) als Kunststoffspritzteil ausgebildet ist und zwischen den Magnetaufnahmekörper (8) und den ersten Gehäusekörper (6) eingespritzt ist.

2. Sensor nach Anspruch 1,
**dadurch gekennzeichnet,**
**daß** sich an dem der in den Strömungskanal ragenden Stirnfläche (30) abgewandten Ende (24) des ersten Gehäusekörpers (6) ein zweiter Gehäusekörper (12) aus einem Kunststoff anschließt, welcher als Kunststoffspritzteil mit zumindest einem Teil (20; 20a) des Isolationskörpers (20; 20a, 20b, 20c) einstückig ausgebildet ist.

3. Sensor nach Anspruch 2,
**dadurch gekennzeichnet,**
**daß** der zweite Gehäusekörper (12) einen Anschlußraum (13) umschließt, in welchem elektrische Verbindungsleitungen (5, 7) mit dem ersten Gehäusekörper (6) und dem Magnetaufnahmekörper (8) elektrisch leitend verbunden sind.

4. Sensor nach Anspruch 2 oder 3,
**dadurch gekennzeichnet,**
**daß** der zweite Gehäusekörper (12) gegenüber dem ersten Gehäusekörper (6) mittels eines ersten Dichtrings (41) abgedichtet ist, der in eine ringförmige Ausnehmung (40) des ersten Gehäusekörpers (6) eingelegt ist und an welchem der als Kunststoffspritzteil ausgebildete zweite Gehäusekörper (12) dicht anliegt, indem ein Vorsprung (44) des zweiten Gehäusekörpers (12) die Ausnehmung (40) des ersten Gehäusekörpers (6) zusammen mit dem ersten Dichtring (41) vollständig ausfüllt.

5. Sensor nach Anspruch 4,
**dadurch gekennzeichnet,**
**daß** der Befestigungsabschnitt des ersten Gehäusekörpers (6) ein Gewinde (18) ist und daß der zweite Gehäusekörper (12) an einer Mantelfläche eine Angriffsfläche (19) aufweist, an welcher ein Werkzeug zum Einschrauben des Sensors (1) in die Wandung des Strömungskanals angreifen kann.

6. Sensor nach einem der Ansprüche 1 bis 5,
**dadurch gekennzeichnet,**
**daß** der erste Gehäusekörper (6) und der Magnetaufnahmekörper (8) Nuten (21, 21a, 21b, 14) und/oder Vorsprünge aufweisen, in welche an dem Isolationskörper ausgebildete Vorsprünge und/oder Nuten eingreifen.

7. Sensor nach einem der Ansprüche 1 bis 6,
**dadurch gekennzeichnet,**
**daß** der Isolationskörper (20a, 20b, 20c) als mehrteiliges Bauteil ausgebildet ist, das einen mit dem Fluid (16) in Kontakt stehenden Isolationsring (20b), eine sich daran axial anschließende Isolationshülse (20a) und einen zwischen dem Isolationsring (20b) und der Isolationshülse (20a) angeordneten zweiten Dichtring (20c) umfaßt.

8. Sensor nach Anspruch 2 und 7,
**dadurch gekennzeichnet,**
**daß** der zweite Gehäusekörper (12) und die Isolationshülse (20a) als einteiliges Kunststoffspritzteil ausgebildet sind.

9. Sensor nach Anspruch 7 oder 8,
**dadurch gekennzeichnet,**
**daß** der Isolationsring (20b) axiale Nuten oder Bohrungen aufweist.

10. Sensor nach einem der Ansprüche 1 bis 11,
**dadurch gekennzeichnet,**
**daß** in den Isolationskörper (20; 20a, 20b, 20c) an seiner in den Strömungskanal ragenden Stirnfläche (28) ein metallischer Ring einsetzbar ist, der die Stirnfläche (28) des Isolationskörper (20; 20a, 20b, 20c) verschmälert.

11. Sensor nach einem der Ansprüche 2 bis 5,
**dadurch gekennzeichnet,**
**daß** der erste Gehäusekörper (6) und der zweite Gehäusekörper (12) durch ein Rändel oder eine Verzahnung miteinander kraftschlüssig verbunden sind.

## Claims

1. Sensor (1) for detecting the contamination by metal particles (54) of a fluid (16) flowing in a flow channel,
with a magnet (17) connected to a first pole (52) of an electric terminal,
an insulating body (20; 20a, 20b, 20c) radially surrounding the magnet (17) and
a metal first housing body (6) radially surrounding the insulating body (20; 20a, 20b, 20c), electrically insulated from the magnet (17) by the insulating body (20; 20a, 20b, 20c) and connected to a second pole (53) of an electric terminal and the outer casing surface of which has a fastening section (18) for fastening the sensor (1) to a wall of the flow channel,
wherein the end faces (27, 28, 30) of the magnet (17), the insulating body (20; 20a, 20b, 20c) and the first housing body (6) project into the flow channel in the state fastened to the flow channel and the electrical resistance between the poles (52, 53) of the electric terminal is reduced by metal particles (54) collecting on the magnet (17) and bridging the insulating body (20; 20a, 20b, 20c),
wherein the magnet (17) is enclosed in a metal magnet accommodating body (8) and
wherein the insulating body (20) or an insulating sleeve (20a) of a multi-part insulating body (20a, 20b, 20c) is constructed as a plastics material moulded part and is injected between the magnet accommodating body (8) and the first housing body (6).

2. Sensor according to claim 1, **characterised in that** adjoining the end (24) of the first housing body (6) facing away from the end face (30) projecting into the flow channel is a second housing body (12) made of a plastics material and constructed as a plastics material moulded part in one piece with at least one part (20; 20a) of the insulating body (20; 20a, 20b, 20c).

3. Sensor according to claim 2, **characterised in that** the second housing body (12) surrounds a connecting space (13) in which electrical connecting lines (5, 7) are connected as electrically conducting to the first housing body (6) and the magnet accommodating body (8).

4. Sensor according to claim 2 or 3, **characterised in that** the second housing body (12) is sealed against the first housing body (6) by means of a first sealing ring (41), which is placed into a ring-shaped recess (40) in the first housing body (6) and against which the second housing body (12) constructed as a plastics material moulded part fits closely **in that** a projection (44) of the second housing body (12) together with the first sealing ring (41) completely fills the recess (40) of the first housing body (6).

5. Sensor according to claim 4, **characterised in that** the fastening section of the first housing body (6) is a thread (18) and the second housing body (12) has on an outer casing surface a working surface (19) to which a tool for screwing the sensor (1) into the wall of the flow channel can be applied.

6. Sensor according to one of claims 1 to 5, **characterised in that** the first housing body (6) and the magnet accommodating body (8) have grooves (21, 21a, 21b, 14) and/or projections in which projections and/or grooves constructed on the insulating body engage.

7. Sensor according to one of claims 1 to 6, **characterised in that** the insulating body (20a, 20b, 20c) is constructed as a multi-part component comprising an insulating ring (20b) in contact with the fluid (16), an insulating sleeve (20a) adjoining this axially and a second sealing ring (20c) arranged between the insulating ring (20b) and the insulating sleeve (20a).

8. Sensor according to claim 2 and 7, **characterised in that** the second housing body (12) and the insulating sleeve (20a) are constructed as a one-part plastics material moulded part.

9. Sensor according to claim 7 or 8, **characterised in that** the insulating ring (20b) has axial grooves or holes.

10. Sensor according to one of claims 1 to 11, **characterised in that** a metal ring which narrows the end face (28) of the insulation body (20; 20a, 20b, 20c) can be inserted into the insulating body (20; 20a, 20b, 20c) on its end face (28) projecting into the flow channel.

11. Sensor according to one of claims 2 to 5, **characterised in that** the first housing body (6) and the second housing body (12) are non-positively connected to one another by knurling or toothing.

## Revendications

1. Capteur (1) pour détecter la contamination d'un fluide (16) avec des particules métalliques (54), s'écoulant dans un canal d'écoulement avec
un aimant (17), lequel est relié à un premier pôle (52) d'un raccord électrique,
un corps d'isolation (20 ; 20a, 20b, 20c) entourant de manière radiale l'aimant (17), et
un premier corps de boîtier métallique (6), entourant de manière radiale le corps d'isolation (20 ; 20a, 20b, 20c), corps de boîtier qui est isolé électriquement de l'aimant (17) par le corps d'isolation (20 ; 20a, 20b, 20c) et est relié à un second pôle (53) d'un raccord électrique et dont la surface d'enveloppe comporte un tronçon de fixation (18) pour fixer le capteur (1) à une paroi du canal d'écoulement,
dans lequel les surfaces frontales (27, 28, 30) de l'aimant (17), du corps d'isolation (20 ; 20a, 20b, 20c) et du premier corps de boîtier (6) dépassent dans le canal d'écoulement dans un état fixé au canal d'écoulement et la résistance électrique entre les pôles (52, 53) du raccord électrique se réduit du fait que des particules métalliques (54) se regroupent sur l'aimant (17) en dérivant le corps d'isolation (20 ; 20a, 20b, 20c),
dans lequel l'aimant (17) est logé dans un corps métallique de réception d'aimant (8), et
dans lequel le corps d'isolation (20) ou un manchon d'isolation (20a) d'un corps d'isolation en plusieurs parties (20a, 20b, 20c) est conçu comme pièce en matière synthétique injectée et est injecté entre le corps de réception d'aimant (8) et le premier corps de boîtier (6).

2. Capteur selon la revendication 1, **caractérisé en ce qu'**à l'extrémité (24) du premier corps de boîtier (6), opposée à la surface frontale (30) dépassant dans le canal d'écoulement, se raccorde un second corps de boîtier (12) en matière synthétique, lequel est conçu en une seule partie comme pièce en matière synthétique injectée avec au moins une partie (20 ; 20a) du corps d'isolation (20 ; 20a, 20b, 20c).

3. Capteur selon la revendication 2, **caractérisé en ce que** le second corps de boîtier (12) enferme un espace de raccord (13), dans lequel des circuits de liaison électriques (5, 7) sont reliés de manière à conduire l'électricité au premier corps de boîtier (6) et au corps de réception d'aimant (8).

4. Capteur selon la revendication 2 ou 3, **caractérisé en ce que** le second corps de boîtier (12) est étanchéisé par rapport au premier corps de boîtier (6) au moyen d'une première bague d'étanchéité (41), qui est placée dans un premier évidement circulaire (40) du premier corps de boîtier (6) et contre lequel s'appuie étroitement le second corps de boîtier (12) conçu comme pièce en matière synthétique injectée alors qu'une avancée (44) du second corps de boîtier (12) remplit totalement l'évidement (40) du premier corps de boîtier (6) conjointement avec la première bague d'étanchéité (41).

5. Capteur selon la revendication 4, **caractérisé en ce que** le tronçon de fixation du premier corps de boîtier (6) est un filetage (18) et **en ce que** le second corps de boîtier (12) comporte sur une surface d'enveloppe une surface d'application (19), sur laquelle un outil peut s'appliquer pour visser le capteur (1) dans la paroi du canal d'écoulement.

6. Capteur selon l'une quelconque des revendications 1 à 5, **caractérisé en ce que** le premier corps de boîtier (6) et le corps de réception d'aimant (8) comportent des rainures (21, 21a, 21b, 14) et/ou des avancées dans lesquelles s'engrènent des rainures et/ou des avancées conçues sur le corps d'isolation.

7. Capteur selon l'une quelconque des revendications 1 à 6, **caractérisé en ce que** le corps d'isolation (20a, 20b, 20c) est conçu comme pièce de construction en plusieurs parties, qui comprend une bague d'isolation (20b) en contact avec le fluide (16), un manchon d'isolation (20a) s'y raccordant de manière axiale et une seconde bague d'étanchéité (20c) disposée entre la bague d'isolation (20b) et le manchon d'isolation (20a).

8. Capteur selon la revendication 2 et 7, **caractérisé en ce que** le second corps de boîtier (12) et le manchon d'isolation (20a) sont conçus comme pièce injectée en matière synthétique d'une seule partie.

9. Capteur selon la revendication 7 ou 8, **caractérisé en ce que** la bague d'isolation (20b) comporte des rainures ou des forages axiaux.

10. Capteur selon l'une quelconque des revendications 1 à 9, **caractérisé en ce que** dans le corps d'isolation (20 ; 20a, 20b, 20c), au niveau de sa surface frontale (28) dépassant dans le canal d'écoulement, peut être placée une bague métallique qui rend la surface frontale (28) du corps d'isolation (20 ; 20a, 20b, 20c) plus étroite.

11. Capteur selon l'une quelconque des revendications 2 à 5, **caractérisé en ce que** le premier corps de boîtier (6) et le second corps de boîtier (12) sont assemblés l'un à l'autre grâce à un crantage ou une denture, à force.
